# EUROPEAN PATENT APPLICATION

(11) **EP 2 594 237 A2**
(43) Date of publication of application: **22.05.2013**
(21) Application number: 13154984.2
(22) Date of filing: 25.06.2008
(51) Int. Cl.: A61F 13/15, A61F 13/56

(54) **Attachment pattern for undergarment attached absorbent articles**

(30) Priority: 30.08.2007 US 847781
(62) Divisional of application: 08776514.5
(71) Applicant: KIMBERLY-CLARK WORLDWIDE, INC., Neenah, WI 54956 (US)
(72) Inventor: GOERG-WOOD, Kristen Ann, Sherwood, WI Wisconsin 54169 (US); MICHALSKI, Anthony James, Menomonee Falls, WI Wisconsin 53051 (US)
(74) Representative: Davies, Christopher Robert

(57) **Abstract**

An adhesive pattern for use in absorbent feminine care articles is generally disclosed. The adhesive is applied in a specific pattern to removably secure the article to the crotch portion of an undergarment during use. The use of a particular adhesive pattern has been found by the present inventors to improve performance of the undergarment attached absorbent article during use. The pattern includes separating the adhesive applied to the front portion, the middle portion, and the back portion at the fold lines of the undergarment attached absorbent article that are substantially free from adhesive. This break in the machine direction (i.e. the X direction defining the length of the article) of the undergarment attached absorbent article inhibits "zippering" of the adhesive. Additionally, the middle portion of the undergarment attached absorbent article contains a specifically placed adhesive to enhance the performance and comfort of the undergarment attached absorbent article during use.

## Description

### Background of the Invention

Undergarment attached absorbent articles, including absorbent feminine care articles (e.g., sanitary napkins, panty liners, labial pads, and other types of catamenial devices) and incontinence pads, are used to absorb menses and other body fluids. These absorbent products are used for light incontinence purposes or during a women's menstrual cycle. Generally, undergarment attached absorbent articles are adhered, or otherwise attached, to the undergarment in the crotch region.

Adhesion between the absorbent feminine care article and undergarment (e.g., panties) is currently the number one consumer complaint across pads and panty liners. During use, absorbent feminine care articles are subjected to various forces that strain the adherence between the absorbent feminine care article and the undergarment. For example, lateral forces are applied along the edges of the absorbent feminine care article when the article is initially placed in the undergarment as it conforms to the anatomical contours of the body. Also, movements of the wearer (e.g., walking, sitting, etc.) subject the absorbent feminine care article to applied forces such as sheer stress, torque, and compression forces.

Current garment adhesive patterns, which typically include a continuous adhesive pattern completely covering the side of the garment or solid adhesive bands extending in the machine direction across the entire length of the article, allow "zippering" effect of the adhesive while being worn. The "zippering" effect occurs when one end of the garment becomes loose and "zippers" loose throughout the length of the garment. Additionally, during use, current pads tend to bunch (i.e., form wrinkles in the MD or CD direction of the article) and/or twist without staying in place.

One method to reduce the amount of bunching, twisting, or zippering of the pad during use is to increase the adhesion by using stronger adhesives or more adhesive (e.g., increased add-on amounts). However, it is desirable that the absorbent feminine care article remain removably adhered to the undergarments in order to allow the article to be readily removed when desired. Likewise, increasing the strength or add-on of the adhesive utilized in the article could damage the undergarment when the article is removed.

Thus, a need exists to improve the attachment of an absorbent feminine care article to undergarments during use, while still allowing the article to be readily removable when desired.

### Summary of the Invention

In accordance with one embodiment of the present invention, an undergarment attached absorbent article is disclosed that comprises a liquid permeable topsheet, a generally liquid impermeable backsheet, and an absorbent core positioned between the backsheet and the topsheet. The undergarment attached absorbent article defines a first fold line and a second fold line oriented in the Y direction and separating the article into a front portion, a middle portion, and a back portion, wherein the middle portion defines a middle width in the Y direction and a middle length in the X direction. The undergarment attached absorbent article can have a total thickness measured in a Z direction perpendicular to both the X and Y directions of less than about 5 millimeters.

An attachment mechanism is positioned onto the outer surface of the liquid impermeable backsheet in a manner such that the outer surface of the liquid impermeable backsheet is substantially free of any attachment mechanism about each fold line. The attachment mechanism can be an adhesive, a mechanical attachment mechanism (e.g., hooks in a traditional hook and loop fastener), and combinations thereof. The attachment mechanism is positioned in a first pattern on the front portion of the article and a second pattern to the back portion. Both the first pattern and the second pattern have breaks in the Y direction that are substantially free of any attachment mechanism.

In one embodiment, the first pattern and the second pattern can each comprise from 5 to 8 adhesive bands extending in the X direction. For example, each adhesive band can have a length in the Y direction of from about 50% to about 95% of a total length of the front portion and the back portion, respectively, as measured from an edge of the undergarment attached absorbent article to the respective fold line. Each adhesive band can have a width in the X direction of from about 5% to about 15% of a total width of the front portion and the back portion, respectfully, measured from a longitudinal side edge of the undergarment attached absorbent article to another longitudinal side edge of the undergarment attached absorbent article.

Alternatively, the first pattern and the second pattern can each include a row of adhesive dots positioned along each longitudinal side edge of the front and back portions. For instance, the first and second patterns can each further include adhesive dots arranged in an "X" pattern extending through a center area from opposite corners. A centered adhesive dot can be present that is at least about 25% larger in surface area than the other adhesive dots in the top and bottom portions. A set of adhesive dots arranged in a "V" pattern pointed toward the center of the undergarment attached absorbent article can be positioned along the outer edge of each front and back portion.

The attachment mechanism is applied in a third pattern to the middle portion. In one embodiment, the third pattern defines at least one attachment mechanism dot covering less than about 33% of the middle width and the middle length, such that the at least one attachment mechanism dot is positioned within a center third of the middle width and the middle length. Both the outer thirds of the middle width and the middle length are substantially free from any attachment mechanism. In one embodiment, the third pattern consists of a single attachment mechanism dot substantially centered in both the X direction and the Y direction of the middle portion. For example, the single attachment mechanism dot of the third pattern covers about 15% to about 25% of the middle width and the middle length. Alternatively, the attachment mechanism can be applied in the third pattern as a plurality of dots only to a center third of the middle width and the middle length of the middle portion.

In another embodiment, the third pattern defines from 3 to 6 adhesive bands extending in the X direction to have a length of about 50% to about 90% of the middle length. For example, the third pattern can include 3 or 4 adhesive bands positioned within the middle width of the middle portion such that each outer 25% of the middle width is substantially free from adhesive.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figure in which:
Figs. 1-5 depict exemplary attachment patterns suitable for improving the adhesion of an absorbent feminine care article during use;
Figs. 6-11 show adhesive patterns that did not show substantial improvement of adhesion between the absorbent feminine care article and undergarment during use;
Fig. 12 shows a control adhesive pattern which is currently used commercially;
Fig. 13 shows an adhesive pattern used as a negative control; and

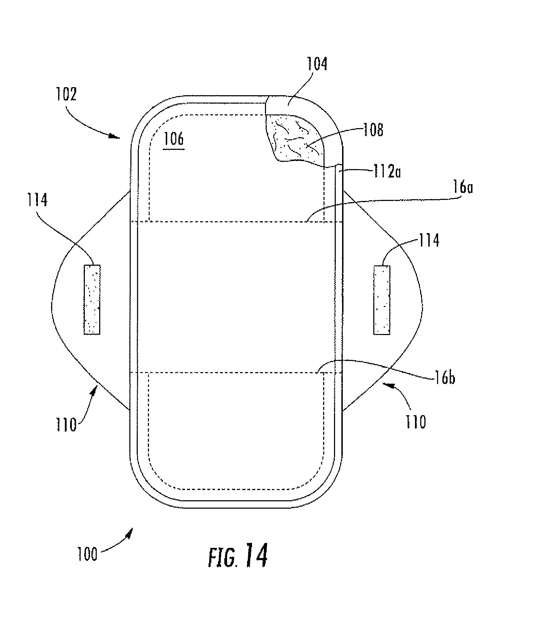

Fig. 14 depicts an exemplary construction of an absorbent feminine care article.

Repeat use of references characters in the present specification and drawing is intended to represent same or analogous features or elements of the invention.

### Detailed Description of Representative Embodiments

Reference now will be made in detail to various embodiments of the invention, one or more examples of which are set forth below. Each example is provided by way of explanation of the invention, not limitation of the invention. In fact, it will be apparent to those skilled in the art that various modifications and variations may be made in the present invention without departing from the scope or spirit of the invention. For instance, features illustrated or described as part of one embodiment, may be used on another embodiment to yield a still further embodiment. Thus, it is intended that the present invention covers such modifications and variations as come within the scope of the appended claims and their equivalents.

Generally speaking, the present invention is directed to an attachment pattern for use in undergarment attached absorbent articles, such as sanitary napkins, panty-liners, labial pads, certain incontinence articles, etc. More specifically, an attachment mechanism, such as an adhesive, is applied in a specific pattern to removably secure the article to the crotch portion of an undergarment during use. The use of a particular attachment pattern has been found by the present inventors to improve performance of the absorbent feminine care article during use.

Additionally, the present inventors have found that the described attachment pattern is particularly suitable for use with ultra-thin, flexible absorbent feminine care articles. These ultra-thin articles have a thickness of less than about 5 millimeters (mm), such as from about 2 mm to about 4 mm. These ultra-thin absorbent feminine care articles are extremely flexible. However, the ease of movement also presents an increase in forces asserted on the attachment mechanism holding the absorbent feminine care article to the undergarment.

Although the following description is directed to an adhesive applied to an absorbent feminine care article, it should be understood that the description also relates to other types of attachment mechanisms and combinations of attachment mechanisms. For example, instead of an adhesive being applied to the backsheet of the undergarment attached absorbent article, a "hook"-like mechanical attachment mechanism can be utilized, such as disclosed in U.S. Publication No. 2005/0124960 of Ruman, which is hereby incorporated by reference in its entirety. Conventional undergarment materials, such as natural or synthetic fiber woven or nonwoven materials, inherently function as a "loop" or "hook compatible material" when engaged by conventional hook or micro-hook materials and, thus, the backsheet (i.e., baffle) hook material releasably attaches to the undergarment. The hook material constitutes the male component of conventional hook-and-loop mechanical fastening systems and cooperates with a "loop" or loop-like material to define a releasable and re-attachable fastening system. Any number of commercially available and conventional micro-hook materials used in absorbent articles, including diaper attachment tabs, etc., may be used in the present invention. Conventional systems are, for example, available under the VELCRO trademark. The hook element may be provided by a single-prong hook configuration, a multiple-prong hook configuration or by a generally continuous, expanded-head configuration, such as provided by a mushroom-head type of hook element. The many arrangements and variations of such fastener systems are collectively known in the art as hook-and-loop fasteners.

Likewise, it should be appreciated that the invention is in no way limited to sanitary napkins in particular, or to feminine care articles in general, even though the invention has particular usefulness for feminine care articles. One skilled in the art will readily understand the adaptability of the invention to other personal care and health care articles, such as, for example, adult incontinence garments and the like that attach to a wearer's undergarment.

### I. Adhesive Pattern

According to the present invention, the attachment mechanism is positioned onto the undergarment attached absorbent article in a discontinuous manner in both the machine direction (MD) and cross-machine direction (CD). Specifically, the attachment pattern has breaks in the MD at least about the area separating the article into substantially equal thirds in the length (MD) direction (e.g., around the fold lines of the absorbent feminine care article).

In conventional absorbent feminine care articles, two fold lines are present in the CD, which generally separates the absorbent feminine care article into substantially equal thirds: a front portion, a middle portion, and a back portion. However, the presence of the fold lines is not necessarily present in the absorbent feminine care article, depending on the particular packaging and storage of the absorbent feminine care article. As such, the term "fold lines" is used herein to represent lines directed in the CD, whether actually present or not in the final article, that generally separate the absorbent feminine care article into substantially equal thirds: a front portion, a middle portion, and a back portion. Thus, the fold lines can simply represent imaginary lines that separate the absorbent feminine care article into substantially equal thirds.

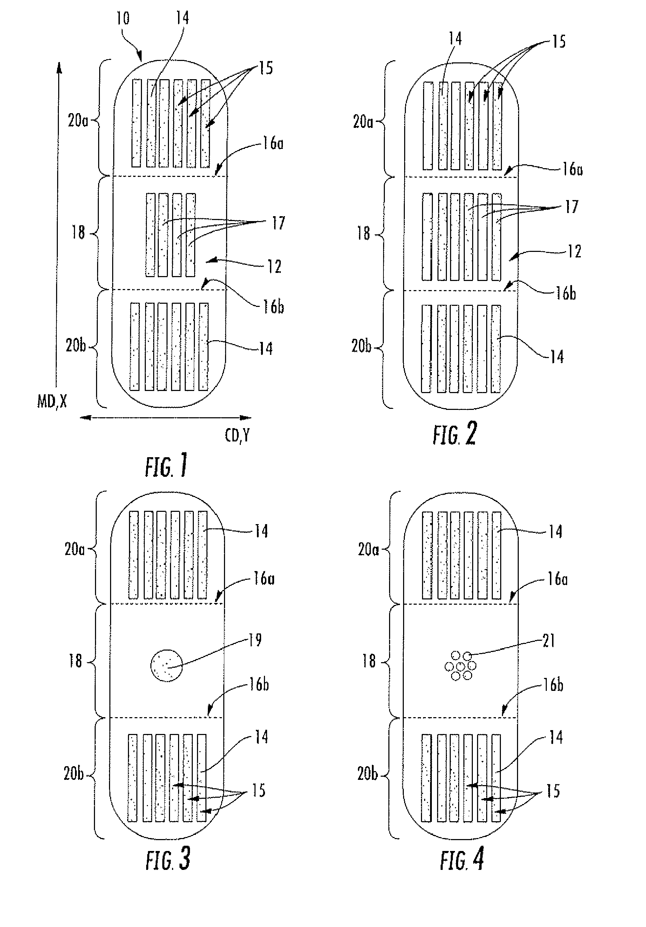

The adhesive pattern is applied to the absorbent feminine care article in a manner that can generally be described by the differences in the front, middle, and back portions separated by the fold lines at substantially equal length thirds (as measured in the MD). For example, referring to Figs. 1-13, the fold lines (16a, 16b) separate the absorbent feminine care article 10 into a middle portion 18 located between a front portion 20a and a back portion 20b.

Movements during use, especially when walking or running, create a twisting, flexing motion of the absorbent feminine care article that strains the adherence of the article to the undergarment, especially at the front and back portions (20a, 20b) of the article 10. Specifically, this twisting, flexing motion creates greater strain on the adhesion bonds areas along the edges of both the front and back portions (20a, 20b) than on the adhesion bonds located in the middle portion 18. Thus, the present adhesion pattern utilizes different adhesion patterns within the middle portion 18 and either of the front or back portions (20a, 20b).

The embodiments described immediately below for the front and back portions (20a, 20b) can be utilized interchangeably with any of the embodiments described for the middle portion 18. However, in all embodiments, the adhesive pattern for the front and back portions (20a, 20b) is separated from the adhesive pattern of the middle portion 18 at a break in adhesive application (i.e., substantially free from adhesive) on the surface 12 at about the fold lines (16a, 16b). This break in adhesive at the fold lines (16a, 16b) prevents a "zippering" effect in the MD during use. For instance, if the adhesive band becomes unattached in either the front and/or back portion (20a, 20b), this failed adhesive does not readily transfer to the adhering pattern of the middle portion.

### A. Adhesive Pattern of Front and Back Portions

According to the present invention, the front and back portions generally have an adhesive pattern with a discontinuous pattern at least in the CD. For example, in one embodiment, the front and back portions can include a plurality of adhesive bands each extending in the MD. As used herein, the term "adhesive band" generally refers to a substantially rectangular strip of adhesive. Referring to Figs. 1-4, the front and back portions (20a, 20b) show the adhesive 14 applied to the surface 12 of the absorbent feminine care article 10 in a plurality of adhesive bands 15, each band extending in the MD. The adhesive bands 15 are separated in the CD by areas of surface 12 that are substantially free from any adhesive.

The size of the adhesive bands can vary relative to the size of the absorbent feminine care article. To ease the application process and create more uniform adherence, it is generally desirable to have each band be substantially the same size and shape in each of the front and back portions (20a, 20b). In one particular embodiment, the bands have a length in the MD extending from about 50% to about 95% of the total length (i.e., the length in the MD from the lateral edge of the absorbent feminine care article to the first fold line (16a, 16b)) of the front and back portions (20a, 20b), such as from about 75% to about 90% of the total length of the front and back portions (20a, 20b) in the MD.

The width of the adhesive bands can also vary as a function of the width of the absorbent feminine care article as well as the number of adhesive bands desired. In particular embodiments, the adhesive bands have substantially the same width and are substantially equally spaced from each other. Also, in a particular embodiment, from about 5 to about 8 adhesive bands are present in each front and back portion (20a, 20b) to provide the maximum adherence during use. The use of about 5 to about 8 adhesive bands can inhibit bunching of the absorbent feminine care article and reduce any "zippering" effect.

When using about 5 to about 8 adhesive bands, the width of each adhesive band can be from about 5% to about 15% of the total width in the CD (i.e., measured from one longitudinal edge of the absorbent feminine care article to the other longitudinal edge) of the absorbent feminine care article, such as from about 5% to about 10%. For example, when 6 adhesive bands are present in each front and back portion (20a, 20b), the width of each adhesive band can be from about 5% to about 15% of the total width in the CD.

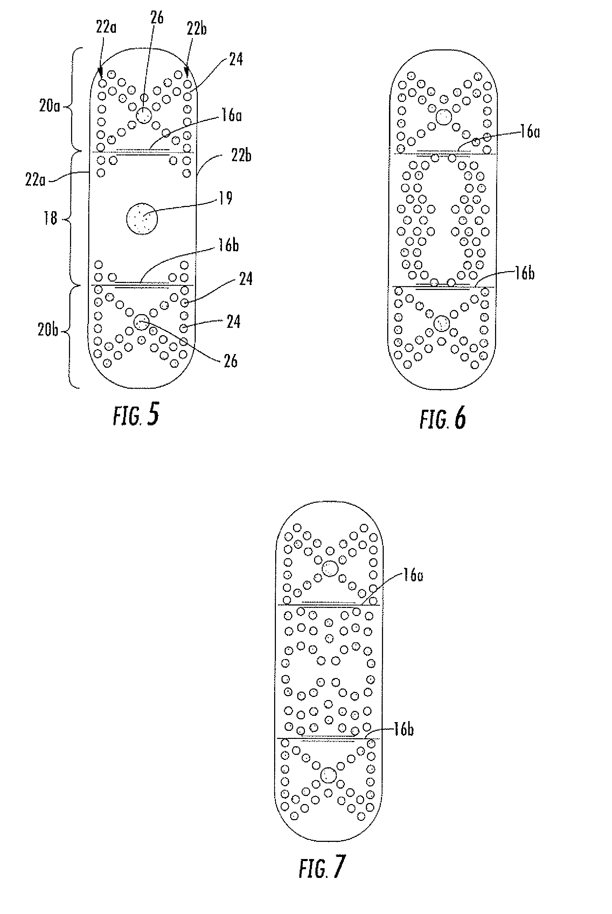

In an alternative embodiment, the adhesive pattern of the front and back portions can be a pattern of a plurality of adhesive dots. Utilizing an pattern of adhesive dots allows for a pattern that is discontinuous in both the MD and CD directions. In this embodiment, the adhesive dots can further inhibit bunching and/or a zippering effect by isolating each adhesive area from another. A particular embodiment showing an exemplary arrangement of adhesive dots will now be described with reference to Fig. 5, although other patterns of adhesive dots can be utilized, as long as there is a break in both the CD and MD directions.

Fig. 5, for example, depicts the front and back portions (20a, 20b) having a row (22a, 22b) of adhesive dots 24 positioned along the longitudinal side edges (24a, 24b) of the surface 12. Although Fig. 5 depicts 8 adhesive dots 24 positioned in the row of adhesive dots along the longitudinal side edges, in other embodiments, 4 to 12 adhesive dots can be positioned in the row of adhesive dots along the longitudinal side edges of the surface 12, such as from 6 to about 10 adhesive dots. Additionally, the front and back portions (20a, 20b) have adhesive dots arranged in an "X" pattern extending through the center area from opposite corners of the front and back portions. For example, Fig. 5 depicts a centered adhesive dot 26 that is slightly larger than the other adhesive dots and 3 adhesive dots extending from the center to each corner of the front and back portions to form the legs of the "X" and connect the "X" to the longitudinal rows of adhesive dots. In other embodiments, 1 to 6 adhesive dots can be utilized to form the legs of the "X". Finally, a set of adhesive dots arranged in a "V" pattern pointed toward the center of the absorbent feminine care article is positioned along the outer edge of each front and back portion (20a, 20b). The set of adhesive dots arranged in a "V" pattern provides extra strength for the attachment of the lateral edges of the absorbent feminine care article, where the greatest amount of stress is generally located during use.

The size of the adhesive dots utilized on the front and back portions (20a, 20b) of the absorbent feminine care article can vary according to the surface area of the absorbent feminine care article. However, in most embodiments, the size of the adhesive dots, except for a center adhesive dot, can be substantially uniform. In one particular embodiment, the adhesive dot positioned substantially in the center of each of the front and back portions (20a, 20b) is larger than the rest of the adhesive dots in the front and back portions (20a, 20b). This larger center dot can be at least about 25% larger in surface area than the other adhesive dots in the front and back portions (20a, 20b), such as from about 50% to about 200% larger. Although the adhesive dots are circular in exemplary Fig. 5, any other shape of adhesive "dots" can be utilized (e.g., squares, diamonds, ovals, etc.).

The front and back portions have substantially identical adhesive patterns in most embodiments, although not a requirement. No matter the specific discontinuous adhesive pattern in the CD of the front and back portions, the adhesive 14 generally covers from about 40% to about 80% of the total surface area of both the front and back portions, such as from about 50% to about 75%. This particular amount of coverage of the surface area in the front and back portions has been found to provide sufficient attachment strength during use of the absorbent feminine care article 10 while still minimizing the amount of adhesive required to be applied to the surface.

### B. Adhesive Pattern of Middle Portion

Stress forces occur along the outer longitudinal edges in the MD of the middle portion 18 during use. Conversely, the center area of the middle portion 18, measured in both the MD and CD, is subjected to the least amount of stress forces throughout the entire absorbent feminine care article 10. However, the overall stress forces in the middle portion 18 are less pronounced than those in the front and back portions (20a, 20b). Thus, an opportunity exists to decrease the amount of adhesive applied in the middle portion, which can lead to decreased material costs (i.e., less adhesive used).

According to one embodiment, less surface area of adhesive is generally used in the middle portion 18 than in either the front or back portion (20a, 20b). For example, adhesive can be applied to cover the middle portion at a surface area amount that is less than 80% of the total adhesive surface area in either the front or back portion (20a, 20b), such as less than about 66%.

The present inventors have surprisingly discovered that the concentration of adhesive toward the center area of the middle portion 18 improves the performance of the absorbent feminine care article 10. Additionally, it has been surprisingly discovered that the absence of adhesive along the outer longitudinal edges of the surface 12 improves the performance of the absorbent feminine care article 10. This discovery is generally contrary to the known conventional reasoning that the adhesive must be applied to those areas where the stress forces are the greatest. Specifically, the application of the adhesive to the center areas of the middle portion 18 has been found to reduce bunching of the absorbent feminine care article, while still allowing for greater flexibility during use.

In one embodiment, the adhesive is applied in adhesive bands extending in the MD. For example, referring to Figs. 1 and 2, a surface 12 of an absorbent feminine care article 10 is shown. An adhesive 14 is applied on the surface 12 in bands extending in the MD of the absorbent feminine care article 10. In Fig. 1, the adhesive bands 17 are applied in the center area of the CD, leaving the longitudingal side edges of the middle portion 18 substantially free from adhesive. The adhesive bands 17 extending in the MD are separated in the CD by areas that are substantially free from adhesive.

The size of the adhesive bands 17 can vary relative to the size of the absorbent feminine care article. To ease the application process and create more uniform adherence, it is generally desirable to have each band be substantially the same size and shape in the middle portion 18. In one particular embodiment, the bands have a length in the MD extending from about 90% to about 50% of the total length (i.e., the length in the MD from fold line 16a to fold line 16b) of the middle portion 18, such as from about 60% to about 80% of the total length of the middle portion 18 in the MD.

The width of the adhesive bands 17 can also vary as a function of the width of the absorbent feminine care article as well as the number of adhesive bands desired. In particular embodiments, the adhesive bands have substantially the same width and are substantially equally spaced from each other. Also, in a particular embodiment, from about 3 to about 6 adhesive bands are present in the middle portion 18 to provide the maximum adherence during use. The use of about 3 to about 6 adhesive bands can inhibit bunching of the absorbent feminine care article and reduce any "zippering" effect.

When using about 3 to about 6 adhesive bands, the width of each adhesive band can be from about 5% to about 15% of the total width in the CD (i.e., measured from one longitudinal edge of the absorbent feminine care article to the other longitudinal edge) of the absorbent feminine care article, such as from about 5% to about 10%. For example, when 6 adhesive bands are present in each front and back portion (20a, 20b), the width of each adhesive band can be from about 5% to about 15% of the total width in the CD.

However, in the embodiment having adhesive bands 15 in both the front and back portions (20a, 20b) as well as the middle portion 18, the number of adhesive bands in the middle portion 18 can be fewer than the number of adhesive bands in the front and back portions (20a, 20b). For example, referring to Fig. 1, four adhesive bands are present in the middle portion 18, while six adhesive bands are present in the front and back portions (20a, 20b). This particular configuration allows for the longitudinal side edges of the middle portion 18 to be freer than the longitudinal side edges of the front and back portions (20a, 20b).

In an alternative embodiment, the adhesive can be applied only to the center portion of the middle portion (i.e., only to the middle third of the middle portion in both the MD and CD). The adhesive can be concentrated within the middle portion as a single adhesive dot or as a plurality of dots.

Referring to Figs. 3 and 5, for example, only a single adhesive dot 19 is positioned substantially in the center of the middle portion 18, as measured in both the MD and CD. In this embodiment, the size of the adhesive dot 19 can vary according to the size of the middle portion 18. Particularly, the centered adhesive dot 19 can cover less than about 33% of the middle width of the middle portion 18 (i.e., less than about the center third of the width of the middle portion 18 measured in the CD from one longitudinal side edge to the other longitudinal side edge) and less than about 33% of the middle length of the middle portion 18 (i.e., less than about the center third of the length of the middle portion 18 measured in the MD from fold line 16a to fold line 16b). For example, the centerly positioned adhesive dot 19 can cover from about 15% to about 25% of the middle width of the middle portion 18 and from about 15% to about 25% of the middle length of the middle portion 18. As such, at least the outer thirds of the middle portion 18, as measured in both the MD and CD directions, is substantially free from adhesive, even though these areas of the middle portion 18 are subjected to more stress forces than the center thirds of each direction. It has been surprisingly found by the present inventors that the absence of adhesive in at least the outer thirds of the middle portion 18 enables the absorbent feminine care article 10 to freely flex with the movement of the wearer without sacrificing the performance of the article. Although the adhesive dot 19 is circular in exemplary Figs. 3 and 5, any other shape of adhesive "dots" can be utilized (e.g., squares, diamonds, ovals, etc.), as long as the overall size is in conformance with the present description.

Alternatively, referring to Fig. 4, a plurality of adhesive dots 21 can be positioned within the center region of middle portion 18. As shown, seven adhesive dots 21 are positioned within the center thirds of the middle portion 18, as measured in both the MD and CD. However, any number, configuration, size, or shape of adhesive dots 21 can be utilized, as long as the outer thirds of the middle portion 18 in both the MD and CD are substantially free from adhesive.

In one particular embodiment, the attachment mechanism utilized in the middle portion 18 can be different than then attachment mechanism utilized in the front and back portions (20a, 20b). For example, a hook-like mechanical attachment mechanism can be utilized in the middle portion 18, while a stronger attachment mechanism, such as an adhesive, is utilized in the front and back portions (20a, 20b).

### II. Absorbent Article

As is well known in the art, the absorbent feminine care article may be provided with adhesives (e.g., pressure-sensitive adhesives) that help removably secure the article to the crotch portion of an undergarment. Suitable pressure-sensitive adhesives, for instance, may include acrylic adhesives, natural rubber adhesives, tackified block copolymer adhesives, polyvinyl acetate adhesives, ethylene vinyl acetate adhesives, silicone adhesives, polyurethane adhesives, thermosettable pressure-sensitive adhesives, such as epoxy acrylate or epoxy polyester pressure-sensitive adhesives, etc. The pressure sensitive adhesives may also include additives such as cross-linking agents, fillers, gases, blowing agents, glass or polymeric microspheres, silica, calcium carbonate fibers, surfactants, and so forth. Particularly suitable adhesives are available commercially under the trade names BOSTIK H2961 and BOSTIK H5400 from Bostic, Inc. (Wauwatosa, WI).

The additives are included in amounts sufficient to affect the desired properties. However, as described above, other types of attachment mechanisms, or combinations of attachment mechanisms, can be utilized in conjunction with the absorbent feminine care article of the present invention.

Certain feminine hygiene products (e.g., sanitary napkins) may have wings or flaps that laterally extend from a central absorbent core and are intended to be folded around the edges of the wearer's panties in the crotch region. The flaps may be provided with an adhesive (e.g., pressure-sensitive adhesive) for affixing the flaps to the underside of the wearer's panties. However, in one embodiment, wings may be omitted from the absorbent feminine care article since the adhesive pattern can improve the overall performance of the article and negate any need for the presence of wings or flaps on the article.

In this regard, various embodiments of an absorbent feminine care article that may be formed according to the present invention will now be described in more detail. For purposes of illustration only, an absorbent feminine care article 100 is shown in Fig. 15 as a sanitary napkin for feminine hygiene. In the illustrated embodiment, the absorbent feminine care article 100 includes a main body portion 102 containing a topsheet (i.e., body-side liner) 104, an outer cover or backsheet (i.e., baffle) 106, an absorbent core 108 positioned between the backsheet 106 and the topsheet 104, and an optional pair of flaps 110 extending from each longitudinal side 112a of the main body portion 102. The topsheet 104 defines a bodyfacing surface of the absorbent article 100. The absorbent core 108 is positioned inward from the outer periphery of the absorbent article 100 and includes a body-facing side positioned adjacent the topsheet 104 and a garment-facing surface positioned adjacent the backsheet 106.

The topsheet 104 is generally designed to contact the body of the user and is liquid-permeable. The topsheet 104 may surround the absorbent core 108 so that it completely encases the absorbent article 100. Alternatively, the topsheet 104 and the backsheet 106 may extend beyond the absorbent core 108 and be peripherally joined together, either entirely or partially, using known techniques. Typically, the topsheet 104 and the backsheet 106 are joined by adhesive bonding, ultrasonic bonding, or any other suitable joining method known in the art. The topsheet 104 is sanitary, clean in appearance, and somewhat opaque to hide bodily discharges collected in and absorbed by the absorbent core 108. The topsheet 104 further exhibits good strike-through and rewet characteristics permitting bodily discharges to rapidly penetrate through the topsheet 104 to the absorbent core 108, but not allow the body fluid to flow back through the topsheet 104 to the skin of the wearer. For example, some suitable materials that may be used for the topsheet 104 include nonwoven materials, perforated thermoplastic films, or combinations thereof. A nonwoven fabric made from polyester, polyethylene, polypropylene, bicomponent, nylon, rayon, or like fibers may be utilized. For instance, a white uniform spunbond material is particularly desirable because the color exhibits good masking properties to hide menses that has passed through it. U.S. Pat. No. 4,801,494 to Datta et al. and U.S. Pat. No. 4,008,026 to Sukiennik, et al. teach various other cover materials that may be used in the present invention.

The topsheet 104 may also contain a plurality of apertures (not shown) formed therethrough to permit body fluid to pass more readily into the absorbent core 108. The apertures may be randomly or uniformly arranged throughout the topsheet 104, or they may be located only in the narrow longitudinal band or strip arranged along the longitudinal axis X--X of the absorbent article 100. The apertures permit rapid penetration of body fluid down into the absorbent core 108. The size, shape, diameter and number of apertures may be varied to suit one's particular needs.

As stated above, the absorbent article also includes a backsheet 106. The backsheet 106 is generally liquid-impermeable and designed to face the inner surface, i.e., the crotch portion of an undergarment (not shown). The backsheet 106 may permit a passage of air or vapor out of the absorbent article 100, while still blocking the passage of liquids. Any liquid-impermeable material may generally be utilized to form the backsheet 106. For example, one suitable material that may be utilized is a microembossed polymeric film, such as polyethylene or polypropylene. In particular embodiments, a polyethylene film is utilized that has a thickness in the range of about 0.2 mils to about 5.0 mils, and particularly between about 0.5 to about 3.0 mils.

The absorbent article 100 also contains an absorbent core 108 positioned between the topsheet 104 and the backsheet 106. The absorbent core 108 may be formed from a single absorbent member or a composite containing separate and distinct absorbent members. It should be understood, however, that any number of absorbent members may be utilized in the present invention. For example, in one embodiment, the absorbent core 108 may contain an intake member (not shown) positioned between the topsheet 104 and a transfer delay member (not shown). The intake member may be made of a material that is capable of rapidly transferring, in the z-direction, body fluid that is delivered to the topsheet 104. The intake member may generally have any shape and/or size desired. In one embodiment, the intake member has a rectangular shape, with a length equal to or less than the overall length of the absorbent article 100, and a width less than the width of the absorbent article 100. For example, a length of between about 150 mm to about 300 mm and a width of between about 10 mm to about 60 mm may be utilized.

Any of a variety of different materials are capable of being used for the intake member to accomplish the above-mentioned functions. The material may be synthetic, cellulosic, or a combination of synthetic and cellulosic materials. For example, airlaid cellulosic tissues may be suitable for use in the intake member. The airlaid cellulosic tissue may have a basis weight ranging from about 10 grams per square meter (gsm) to about 300 gsm, and in some embodiments, between about 100 gsm to about 250 gsm. In one embodiment, the airlaid cellulosic tissue has a basis weight of about 200 gsm. The airlaid tissue may be formed from hardwood and/or softwood fibers. The airlaid tissue has a fine pore structure and provides an excellent wicking capacity, especially for menses.

If desired, a transfer delay member (not shown) may be positioned vertically below the intake member. The transfer delay member may contain a material that is less hydrophilic than the other absorbent members, and may generally be characterized as being substantially hydrophobic. For example, the transfer delay member may be a nonwoven fibrous web composed of a relatively hydrophobic material, such as polypropylene, polyethylene, polyester or the like, and also may be composed of a blend of such materials. One example of a material suitable for the transfer delay member is a spunbond web composed of polypropylene, multilobal fibers. Further examples of suitable transfer delay member materials include spunbond webs composed of polypropylene fibers, which may be round, tri-lobal or poly-lobal in cross-sectional shape and which may be hollow or solid in structure. Typically the webs are bonded, such as by thermal bonding, over about 3% to about 30% of the web area. Other examples of suitable materials that may be used for the transfer delay member are described in U.S. Pat. No. 4,798,603 to Meyer, et al. and U.S. Pat. No. 5,248,309 to Serbiak, et al., which are incorporated herein in their entirety by reference thereto for all purposes. To adjust the performance of the invention, the transfer delay member may also be treated with a selected amount of surfactant to increase its initial wettability.

The transfer delay member may generally have any size, such as a length of about 150 mm to about 300 mm. Typically, the length of the transfer delay member is approximately equal to the length of the absorbent article 100. The transfer delay member may also be equal in width to the intake member, but is typically wider. For example, the width of the transfer delay member may be from between about 50 mm to about 75 mm, and particularly about 48 mm. The transfer delay member typically has a basis weight less than that of the other absorbent members. For example, the basis weight of the transfer delay member is typically less than about 150 grams per square meter (gsm), and in some embodiments, between about 10 gsm to about 100 gsm. In one particular embodiment, the transfer delay member is formed from a spunbonded web having a basis weight of about 30 gsm.

Besides the above-mentioned members, the absorbent core 108 may also include a composite absorbent member (not shown), such as a coform material. In this instance, fluids may be wicked from the transfer delay member into the composite absorbent member. The composite absorbent member may be formed separately from the intake member and/or transfer delay member, or may be formed simultaneously therewith. In one embodiment, for example, the composite absorbent member may be formed on the transfer delay member or intake member, which acts a carrier during the coform process described above.

Regardless of its particular construction, the absorbent article 100 contains an adhesive for securing to an undergarment in the patterns discussed above. Furthermore, each of the optional flaps 110 may also contain an adhesive 114 positioned on the flap 110. The adhesive is applied to the exposed outer surface of the backsheet 106 according to any of the patterns discussed above.

A peelable release liner (not shown), which may also be formed in accordance with the present invention, may cover the adhesive pattern and the adhesive 114 before use. Thus, when a user of the sanitary absorbent article 100 wishes to expose the adhesive pattern and secure the absorbent article 100 to the underside of an undergarment, the user simply peels away the liners. Once removed, the release liners may be disposed, either alone or in conjunction with a used absorbent article. Many feminine hygiene products, for example, are disposed by placing them in a small pouch in which the product is packaged for sale. Various suitable pouch configurations are disclosed in U.S. Patent Nos. 6,716,203 to Sorebo, et al. and 6,380,445 to Moder, et al., as well as U.S. Patent Application Publication No. 2003/0116462 to Sorebo, et al., all of which are incorporated herein in their entirety by reference thereto for all purposes.

Although various embodiments of an absorbent feminine care article have been described above that may incorporate the benefits of the present invention, it should be understood that other configurations are also included within the scope of the present invention.

The present invention may be better understood with reference to the following examples.

### Examples

Sample absorbent feminine care articles were made having the patterns shown in Figs. 5 - 13 using an adhesive. The adhesive used in the following examples is available commercially under the trade name BOSTIK H2961 from Bostik, Inc. (Wauatosa, WI). The adhesive was applied to each sample absorbent feminine care article via the following steps:
1. A solid band of adhesive was prepared between two pieces of peel paper. An adhesive width of 60mm was chosen as adequate coverage width with a 30gsm add-on level.
2. A pattern stencil was made from wide peel paper with holes punched out to match the adhesive pattern to be applied to the absorbent feminine care articles. A hammer and a punch was used to make each stencil. A separate stencil was made for each absorbent feminine care articles. Thus, 10 stencils of each of the 10 patterns shown in Figs. 5 - 13 were made (a total of 100 individual stencils).
3. Each absorbent feminine care article was initially proved without any garment adhesive applied to the outer surface of the backsheet. The absorbent feminine care article was a Sputnik Kotex Ultrathin feminine pad available from Kimberly-Clark, Corp, without the garment adhesive applied to the outer surface of the backsheet.
4. To apply the adhesive to the backsheet, the absorbent feminine care article was placed on a flat surface with the topsheet down so that the outer surface of the backsheet is exposed. The stencil was placed on the exposed surface of the backsheet with the release side of the stencil facing away from the backsheet (i.e., exposed).
5. The two peel paper strips with adhesive between the layers was separated and the non-adhesive side of the two peel stripes was disposed.
6. The peel paper strip was placed adhesive side down over the stencil.
7. A hand roller was used to roll over the adhesive's peel paper to ensure adequate transfer.
8. The top peel paper was slowly removed from the back end of the pad.
9. The stencil was slowly removed from the front end of the pad.
10. A fresh peel paper was placed over the finished product.

In order to test each adhesive pattern, one of each sample was given to 10 non-menstrual, nonincontinent women wearing one randomized absorbent feminine care article per day for 5 hours. After the 5 hour wear time the undergarment was cut off with the absorbent feminine care article still attached. The participants then answered a provided questionnaire and received the next day's supplies (i.e. underwear and absorbent feminine care article).

The questions in the survey were as follows:
1. How active were you while wearing this product?
   a. slightly active (some sitting, some walking, some standing)
   b. moderately active (walking, sitting, standing)
   c. very active (exercising, lift, running, cleaning, etc.)
2. How much did the pad bunch in the center?
   rank on a scale of 1-7 (1 = lots of bunching, 7 = no bunching) 3. How much did the ends of the pad fold over?
   rank on a scale of 1-7 (1 = lots of folding, 7 = no folding) 4. How well did the pad conform to your body during wear?
   rank on a scale of 1-7 (1 = conformed poorly, 7 = conformed well) 5. How comfortable was this product to wear?
   rank on a scale of 1-7 (1 = not comfortable, 7 = very comfortable) 6. How much movement of the pad in the underwear did you notice during use?
   rank on a scale of 1-7 (1 = lots of movement, 7 = no movement) 7. How well did the pad stay-in-place on the panty?
   rank on a scale of 1-7 (1 = did not stay-in-place, 7 = stayed-in-place) 8. Based on wearing this pad for 5 hours, how well do you think this pad would have protected you?
   rank on a scale of 1-7 (1 = not well at all, 7 = very well)

The results of the survey are shown in the following table (Table 1) that ranks each adhesive pattern's performance for each question (best performing = highest rank):

**TABLE 1**

| Rank | Q.2 | Q.3 | Q.4 | Q.5 | Q.6 | Q.7 | Q.8 |
|---|---|---|---|---|---|---|---|
| 1 (Best) | Fig. 9 | Fig. 5 | Fig. 5 | Fig. 5 | Fig. 5 | Fig. 5 | Figs. |
| 2 | Fig. 5 | Fig. 7 | Figs. 7, 12 | Fig. 12 | Fig. 12 | Fig. 6 | 5, 8 |
| 3 | Fig 7 | Fig. 9 | | Figs. 6, 10 | Figs. 6, 9 | Figs. 9, 12 | Fig. 6 |
| 4 | Figs. 6, 11, 12 | Figs. 6, 8, 13 | Figs. 6, 8 | | | | Fig. 7 |
| 5 | | | | Fig. 9 | Fig. 8 | Figs. 7, 8 | Fig. 9 |
| 6 | | | Fig. 9 | Fig. 13 | Figs. 7, 10 | | Fig. 12 |
| 7 | Fig. 8 | Figs. 10, 12 | Figs. 11, 13 | Fig. 7 | | Fig. 10 | Fig. 11 |
| 8 | Fig. 13 | | | Fig. 11 | Fig. 13 | Fig. 13 | Figs. 10, 13 |
| 9 (Worst) | Fig. 10 | Fig. 11 | Fig. 10 | Fig. 8 | Fig. 11 | Fig. 11 | |

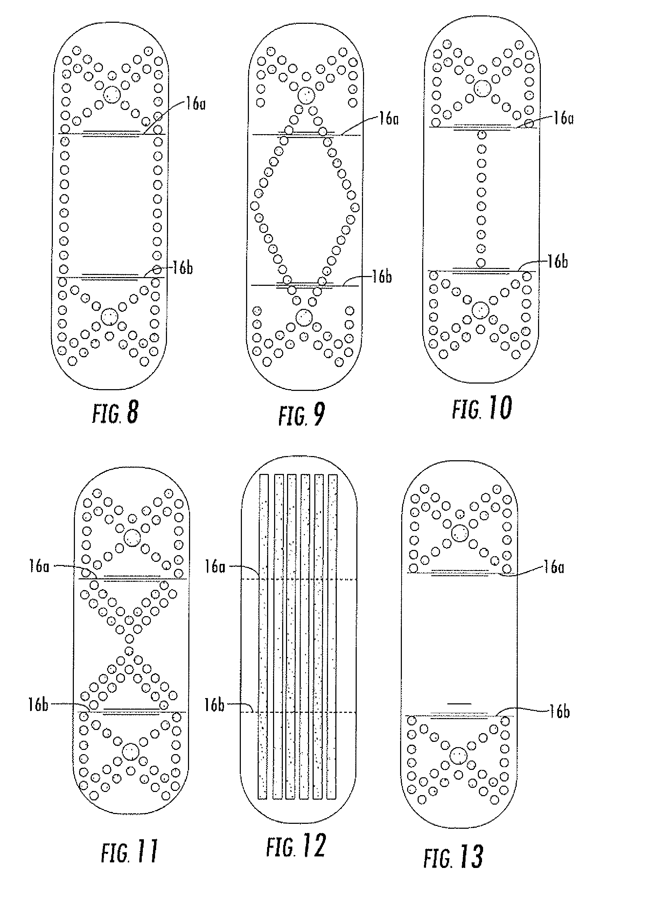

As is shown by Table 1, the adhesive pattern of Fig. 5 surprisingly performed better than all of the other patterns in nearly every category. Equally surprising was that the adhesive pattern of Fig. 10 consistently performed among the worst of the adhesive patterns.

While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. An undergarment attached absorbent article defining an X direction and a Y direction, the undergarment attached absorbent article further defining a first fold line and a second fold line oriented in the Y direction and separating the article into a front portion, a middle portion, and a back portion, wherein the middle portion defines a middle width in the Y direction and a middle length in the X direction measured from the first fold line to the second fold line, the undergarment attached absorbent article comprising
a liquid permeable topsheet;
a liquid impermeable backsheet defining an outer surface;
an absorbent core positioned between the backsheet and the topsheet; and
an adhesive applied to the outer surface of the liquid impermeable backsheet in a manner such that the outer surface of the liquid impermeable backsheet is substantially free of adhesive about each fold line,
wherein the adhesive is applied in a first pattern to the front portion, the first pattern having breaks in the Y direction that are substantially free of adhesive,
wherein the adhesive is applied in a second pattern to the back portion, the second pattern having breaks in the Y direction that are substantially free of adhesive, and
wherein the adhesive is applied in a third pattern to the middle portion, wherein the third pattern defines from 3 to 6 adhesive bands extending in the X direction to have a length of about 50% to about 90% of the middle length.

2. An undergarment attached absorbent article as in claim 1, wherein the first pattern is substantially identical to the second pattern.

3. An undergarment attached absorbent article as in claim 1 or 2, wherein the first pattern and the second pattern each comprise from 5 to 8 adhesive bands extending in the X direction.

4. An undergarment attached absorbent article as in claim 3, wherein each adhesive band has a length in the Y direction of from about 50% to about 95% of a total length of the front portion and the back portion, respectively, as measured from an edge of the undergarment attached absorbent article to the respective fold line and/or wherein each adhesive band has a width in the X direction of from about 5% to about 15% of a total width of the front portion and the back portion, respectfully, measured from a longitudinal side edge of the undergarment attached absorbent article to another longitudinal side edge of the absorbent feminine care article.

5. An undergarment attached absorbent article as in any preceding claim, wherein the third pattern comprises 3 or 4 adhesive bands positioned within the middle width of the middle portion such that each outer 25% of the middle width is substantially free from adhesive.

6. An undergarment attached absorbent article as in any preceding claim, further comprising a release liner releaseably attached to the adhesive.
